# EUROPÄISCHE PATENTSCHRIFT

(11) **EP 1 774 927 B1**
(45) Veröffentlichungstag und Bekanntmachung des Hinweises auf die Patenterteilung: **16.04.2008**
(21) Anmeldenummer: 06121160.3
(22) Anmeldetag: 25.09.2006
(51) Int. Cl.: A61F 2/46, A61F 2/44

(54) **Chirurgisches Instrument zum Entfernen eines Zwischenwirbelimplantats**
Surgical instrument for extraction of an intervertebral implant
Instrument chirurgical servant à extraire un implant intervertébral

(30) Priorität: 14.10.2005 DE 102005050031
(43) Veröffentlichungstag der Anmeldung: 18.04.2007
(73) Patentinhaber: AESCULAP AG & Co. KG, 78532 Tuttlingen (DE)
(72) Erfinder: Schneid, Susanne, 89233 Neu-Ulm (DE)
(74) Vertreter: Hoeger, Stellrecht & Partner Patentanwälte

(56) Entgegenhaltungen:
- US-A1- 2004 059 318
- US-A1- 2004 148 028

## Beschreibung

Die vorliegende Erfindung betrifft ein chirurgisches Instrument zum Entfernen eines Zwischenwirbelimplantats aus einem Zwischenwirbelraum einer menschlichen oder tierischen Wirbelsäule umfassend einen am distalen Ende des Instruments angeordneten Kupplungsteil zum Anlegen an das Zwischenwirbelimplantat in einer Anlegestellung, wobei der Kupplungsteil von der Anlegestellung in eine Kupplungsstellung bringbar ist, in welcher das Zwischenwirbelimplantat kraft- und/oder formschlüssig am Kupplungsteil gehalten ist, wobei der Kupplungsteil mindestens ein erstes Kupplungsglied und mindestens ein zweites Kupplungsglied umfasst, welche mit dem Zwischenwirbelimplantat in der Kupplungsstellung in Eingriff bringbar sind.

Chirurgische Instrumente der eingangs beschriebenen Art werden als sogenannte Revisionsinstrumente in der Wirbelsäulenchirurgie verwendet, um entweder eine Korrektur einer Position des Zwischenwirbelimplantats oder eine Revision vorzunehmen, das heißt, dieses wieder vollständig aus dem Zwischenwirbelraum zu entfernen. Ein Beispiel für ein derartiges chirurgisches Instrument ist in der US 2004/0059318 A1 beschrieben. Der Kupplungsteil des bekannten Instruments umfasst einen Anschlag, welcher am Zwischenwirbelimplantat angelegt werden kann sowie in distaler Richtung vorstehende, federnde Widerhaken, so dass das Zwischenwirbelimplantat zwischen dem Anschlag und den Widerhaken gehalten werden kann. Nachteilig bei einem derartigen Instrument ist, dass mit ihm nur Zwischenwirbelimplantate gleicher Bauart und Größe entfernbar sind.

Es ist daher Aufgabe der vorliegenden Erfindung, ein chirurgisches Instrument der eingangs beschriebenen Art so zu verbessern, dass es auch zum Entfernen unterschiedlicher Zwischenwirbelimplantate unterschiedlicher Größen und Formen verwendet werden kann.

Diese Aufgabe wird bei einem chirurgischen Instrument der eingangs beschriebenen Art erfindungsgemäß dadurch gelöst, dass das mindestens eine erste Kupplungsglied und das mindestens eine zweite Kupplungsglied relativ zueinander derart bewegbar angeordnet sind, dass ein Abstand des mindestens einen ersten Kupplungsglieds und des mindestens einen zweiten Kupplungsglieds in einer Richtung parallel zu einer vom Instrument definierten Längsachse veränderbar ist zum Überführen des Instruments von der Anlegestellung in die Kupplungsstellung und/oder umgekehrt.

Die erfindungsgemäß vorgeschlagene Weiterbildung bekannter Revisionsinstrumente hat den Vorteil, dass Zwischenwirbelimplantate unterschiedlicher Größe und Form mit dem Instrument aus einem Zwischenwirbelraum entfernt werden können. Daher wird für unterschiedliche Zwischenwirbelimplantate nur noch ein Instrument benötigt, das an ein beliebiges Zwischenwirbelimplantat angelegt und anschließend in die Kupplungsstellung überführt werden kann. Dies ist möglich durch Veränderung eines Abstands des mindestens einen ersten Kupplungsglieds und des mindestens einen zweiten Kupplungsglieds. Beispielsweise können das mindestens eine erste Kupplungsglied und das mindestens eine zweite Kupplungsglied in Ausnehmungen des Zwischenwirbelimplantats in der Kupplungsstellung eintauchen und so mit diesem in Eingriff gebracht werden, oder auch nur an dafür vorgesehen Anlageflächen desselben in der Kupplungsstellung anliegen, was auch eine Form des Ineingriffbringens darstellt.

Damit das Instrument von einem Operateur während eines chirurgischen Eingriffs sicher gehalten werden kann, ist vorteilhafterweise an einem proximalen Ende des Instruments ein Griffteil vorgesehen zum Halten desselben.

Um eine Stabilität einer Verbindung des Instruments mit einem Zwischenwirbelimplantat zu verbessern, ist es günstig, wenn zwei erste Kupplungsglieder vorgesehen sind.

Ebenso ist es vorteilhaft, wenn zwei zweite Kupplungsglieder vorgesehen sind.

Um eine individuelle Anpassung des Instruments an unterschiedlich geformte Zwischenwirbelimplantate zu ermöglichen, beispielsweise auch das Anlegen und Verbinden des Instruments mit einem Zwischenwirbelimplantat in einer Richtung oder Ebene, die nicht einer Symmetrieebene des Zwischenwirbelimplantats zugeordnet werden kann, ist es günstig, wenn die zwei zweiten Kupplungsglieder unabhängig voneinander bewegbar sind. Damit kann beispielsweise ein unterschiedlicher Abstand zwischen einem ersten und einem zweiten Kupplungsglied und einem weiteren ersten und einem zweiten Kupplungsglied eingestellt werden.

Gemäß einer bevorzugten Ausführungsform der Erfindung kann vorgesehen sein, dass das mindestens eine erste Kupplungsglied und das mindestens eine zweite Kupplungsglied derart am Instrument angeordnet und gelagert sind, dass sie relativ zueinander parallel oder im Wesentlichen parallel zur Längsachse verschiebbar sind. Dadurch wird der Aufbau des Instruments deutlich vereinfacht und zudem ermöglicht es ein derartiges Instrument, beispielsweise die zweiten Kupplungsglieder an einem Gelenkteil eines Zwischenwirbelimplantats vorbeizuschieben, welches zwischen zwei Anlageplatten desselben beweglich gehalten ist.

Vorteilhaft ist es, wenn das Instrument einen die Längsachse definierenden Schaft umfasst und wenn der Kupplungsteil am distalen Ende oder im Bereich des distalen Endes des Schafts angeordnet ist. Ein einen Schaft aufweisendes Instrument kann beispielsweise auch für einen minimalinvasiven Eingriff verwendet werden, zur Korrektur einer Position des Zwischenwirbelimplantats im Zwischenwirbelraum oder zur Revision.

Damit ein Operateur insbesondere eine Orientierung des mindestens einen ersten und/oder des mindestens einen zweiten Kupplungsglieds auf einfache Weise feststellen kann und um ein Anlegen des Instruments an ein Zwischenwirbelimplantat zu erleichtern, ist es vorteilhaft, wenn das mindestens eine erste Kupplungsglied am distalen Ende oder im Bereich des distalen Endes des Schafts angeordnet ist.

Auf besonders einfache Weise lässt sich das Instrument an ein Zwischenwirbelimplantat anlegen und mit diesem in Eingriff bringen, wenn das mindestens eine erste Kupplungsglied in Form eines in distaler oder im Wesentlichen in distaler Richtung weisenden Kupplungsvorsprungs ausgebildet ist. Beispielsweise kann ein derartiger Kupplungsvorsprung in eine korrespondierende Ausnehmung, insbesondere eine Sacklochbohrung, des Zwischenwirbelimplantats in der Kupplungsstellung eintauchen.

Vorzugsweise ist am Instrument mindestens ein axialer Anschlag vorgesehen zum Begrenzen einer Bewegung des Zwischenwirbelimplantats und des Instruments aufeinander zu. So wird sichergestellt, dass das Instrument in definierter Weise am Zwischenwirbelimplantat angelegt werden kann und sich dieses wiederum bei einer Korrektur der Implantatposition oder einer Revision sicher und definiert abstützen kann.

Ein besonders einfacher Aufbau des Instruments ergibt sich, wenn der mindestens eine axiale Anschlag eine in distaler oder im Wesentlichen in distaler Richtung weisende Anschlagfläche umfasst, an der das Zwischenwirbelimplantat in der Kupplungsstellung mindestens abschnittsweise flächig anliegen kann. Insbesondere kann die Anschlagfläche auch Teil einer am Instrument vorgesehenen Implantataufnahme sein, die korrespondierend zum Zwischenwirbelimplantat ausgebildet ist und in die das Zwischenwirbelimplantat beispielsweise formschlüssig eingeführt werden kann.

Eine besonders gute Kraftübertragung und Abstützung des Instruments und des Zwischenwirbelimplantats relativ zueinander wird erreicht, wenn der mindestens eine axiale Anschlag benachbart oder im Bereich des mindestens einen ersten Kupplungsglieds angeordnet ist.

Die Stabilität des Instruments wird erhöht und das Entfernen des Zwischenwirbelimplantats erleichtert, wenn jedem ersten Kupplungsglied ein axialer Anschlag zugeordnet ist.

Gemäß einer bevorzugten Ausführungsform der Erfindung kann vorgesehen sein, dass das mindestens eine zweite Kupplungsglied eine in Richtung auf den mindestens einen axialen Anschlag hin weisende Klemmfläche umfasst, so dass das Zwischenwirbelimplantat in der Kupplungsstellung zwischen der Klemmfläche und dem mindestens einen axialen Anschlag formschlüssig und/oder klemmend gehalten werden kann. Aufgrund dieser Ausgestaltung kann ein Zwischenwirbelimplantat unterschiedlicher Form und Größe sicher zwischen dem mindestens einen ersten und dem mindestens einen zweiten Kupplungsglied kraft- und/oder formschlüssig gehalten werden.

Um sowohl die Konstruktion als auch die Herstellung des Instruments zu vereinfachen, ist es günstig, wenn das Instrument eine die Längsachse enthaltende Symmetrieebene aufweist.

Vorteilhaft ist es, wenn das mindestens eine zweite Kupplungsglied in der Anlegestellung so weit in distaler Richtung bewegbar ist, dass ein Abstand zwischen dem mindestens einen ersten und dem mindestens einen zweiten Kupplungsglied größer ist als in einer Kupplungsstellung, in welcher das Zwischenwirbelimplantat kraft- und/oder formschlüssig zwischen dem mindestens einen ersten und dem mindestens einen zweiten Kupplungsglied gehalten werden kann. Diese erfindungsgemäße Weiterbildung ermöglicht es, das Instrument so an das Zwischenwirbelimplantat heranzuführen, dass dieses bequem zwischen das mindestens eine erste und das mindestens eine zweite Kupplungsglied eintauchen kann. Erst um das Zwischenwirbelimplantat sicher kraftund/oder formschlüssig am Instrument zu halten, wird ein Abstand zwischen dem mindestens einen ersten und dem mindestens einen zweiten Kupplungsglied verringert, so dass dieses die Kupplungsstellung einnimmt.

Günstig ist es, wenn das mindestens eine zweite Kupplungsglied in der Anlegestellung so weit in proximaler Richtung bewegbar ist, dass es bezogen auf die Längsachse eine Position proximalseitig des mindestens einen ersten Kupplungsglieds einnimmt. So ist es möglich, zunächst nur das mindestens eine erste Kupplungsglied an das Zwischenwirbelimplantat heranzuführen, ohne dass das mindestens eine zweite Kupplungsglied dabei stört. Es nimmt in der beschriebenen Anlegestellung daher eine sogenannte zurückgezogene Position ein.

Besonders einfach läßt sich das Instrument von der Anlegestellung in die Kupplungsstellung überführen und/oder umgekehrt, wenn das mindestens eine zweite Kupplungsglied in einer Ebene quer zur Längsachse bewegbar ist zum Überführen des Instruments von der Anlegestellung in die Kupplungsstellung.

Grundsätzlich wäre es denkbar, dass das mindestens eine zweite Kupplungsglied an einem federnd gelagerten Träger angeordnet ist. Besonders einfach und definiert läßt es sich jedoch von der Anlegestellung in die Kupplungsstellung überführen, wenn das mindestens eine zweite Kupplungsglied um eine Kupplungsgliedlängsachse, die parallel oder im Wesentlichen parallel zur Längsachse verläuft, verdrehbar gelagert ist. So kann das Instrument, anders als bei dem in der US 2004/0059318 A1 beschriebenen Instrument, definiert durch gezieltes Verdrehen des mindestens einen zweiten Kupplungsglieds um die Kupplungsgliedlängsachse von der Anlegestellung in die Kupplungsstellung und umgekehrt überführt werden, so dass das Instrument auch wieder auf einfache Weise vom Zwischenwirbelimplantat entfernt werden kann.

Ein Abstand zwischen dem mindestens einen ersten und dem mindestens einen zweiten Kupplungsglied lässt sich besonders einfach verändern, wenn am Instrument mindestens ein parallel oder im Wesentlichen parallel zur Längsachse verschieblich gelagertes Schub- und Zugglied vorgesehen ist und wenn das mindestens eine zweite Kupplungsglied an einem distalen Ende oder im Bereich desselben am Schub- und Zugglied angeordnet ist und quer oder im Wesentlichen quer zur Längsachse von diesem absteht. Mit dem Schub- und Zugglied kann das mindestens eine zweite Kupplungsglied auf einfache Weise in distaler Richtung vorgeschoben und auch wieder in proximaler Richtung zurückgezogen werden, wodurch ein Abstand zwischen dem mindestens einen ersten und dem mindestens einen zweiten Kupplungsglied veränderbar ist.

Damit das mindestens eine zweite Kupplungsglied auf einfache Weise in einer Ebene quer zur Längsachse bewegt werden kann, ist es günstig, wenn das mindestens eine Schub- und Zugglied eine Kupplungsgliedlängsachse definiert und am Instrument um diese verdrehbar gelagert ist.

Gemäß einer bevorzugten Ausführungsform der Erfindung kann vorgesehen sein, dass am Instrument eine Drehlagerung für das Schub- und Zugglied vorgesehen ist zum Führen einer Verschiebung und/oder einer Verdrehung des Schub- und Zugglieds parallel zur Kupplungsgliedlängsachse. Die vorgesehene Drehlagerung ermöglicht eine einfache Relativbewegung des mindestens einen ersten und des mindestens einen zweiten Kupplungsglieds relativ zueinander sowohl in einer Richtung parallel zur Längsachse als auch in einer Ebene quer zu derselben.

Das Schub- und Zugglied kann auf einfache Weise von einem Operateur bewegt werden, wenn an einem proximalen Ende desselben ein Griffteil vorgesehen ist.

Günstig kann es sein, wenn das mindestens eine Schub- und Zugglied an seinem proximalen Ende oder im Bereich desselben mindestens ein Markierelement aufweist, welches angibt, in welche Richtung das mindestens eine zweite Kupplungsglied weist. Dadurch kann ein Operateur sofort erkennen, ob das Instrument die Anlegestellung oder die Kupplungsstellung einnimmt, da diese beispielsweise durch eine bestimmte Drehstellung des mindestens einen zweiten Kupplungsglied bezogen auf die Kupplungsgliedlängsachse definiert werden kann.

Besonders einfach wird der Aufbau des Instruments, wenn das mindestens eine Markierelement ein quer zur Kupplungsgliedlängsachse abstehender Vorsprung ist, der in dieselbe Richtung weisend angeordnet ist wie das mindestens eine zweite Kupplungsglied. Dadurch wird für einen Operateur direkt erkennbar, in welche Richtung das mindestens eine zweite Kupplungsglied weist, auch wenn ein distales Ende des Instruments in einen menschlichen oder tierischen Körper eintaucht und nicht einsehbar ist.

Um zu verhindern, dass sich ein am Instrument in der Kupplungsstellung kraft- und/oder formschlüssig gehaltenes Zwischenwirbelimplantat von diesem lösen kann, ist es vorteilhaft, wenn am Instrument ein beweglich gelagerter Anschlag vorgesehen ist zum Begrenzen einer Bewegung des mindestens einen zweiten Kupplungsglieds in distaler Richtung. Dadurch wird verhindert, dass sich ein Abstand zwischen dem mindestens einen ersten Kupplungsglied und dem mindestens einen zweiten Kupplungsglied in unbeabsichtigter Weise vergrößert.

Besonders günstig ist es, wenn der beweglich gelagerte Anschlag am Schub- und Zugglied angeordnet ist. Dadurch kann direkt eine Bewegung des Schub- und Zugglieds durch eine Positionsänderung des Anschlags einschränkt werden.

Zur Vereinfachung eines Aufbaus des Instruments kann es günstig sein, wenn das Schub- und Zugglied einen Gewindeabschnitt umfasst und wenn der beweglich gelagerte Anschlag eine zum Gewindeabschnitt korrespondierende und auf diesen aufgeschraubte Mutter umfasst. Beispielsweise kann diese Mutter auch als Kontermutter verwendet werden, um eine Kupplungsstellung, in welcher beispielsweise das Zwischenwirbelimplantat klemmend zwischen dem mindestens einen ersten und dem mindestens einen zweiten Kupplungsglied gehalten ist, zu sichern.

Günstigerweise ist der beweglich gelagerte Anschlag am Schaft angeordnet. Beispielsweise kann der beweglich gelagerte Anschlag mit der Drehlagerung verbunden oder so am Schaft gelagert sein, dass er mit einem Vorsprung am Schub- und Zugglied zusammenwirken kann, beispielsweise einem Drehknopf, der am Schub- und Zugglied angeordnet ist. Dadurch kann insbesondere das Schub- und Zugglied besonders einfach aufgebaut werden.

Vorteilhaft kann es ferner sein, wenn an einem proximalen Ende des Instruments eine quer oder im Wesentlichen quer zur Längsachse orientierte Endplatte angeordnet ist. Diese kann insbesondere beabstandet von einem Griffteil des Instruments angeordnet sein, beispielsweise an einer Verlängerung des Schafts des Instruments über das Griffteil hinaus. Die Endplatte erleichtert insbesondere das Entfernen des Zwischenwirbelimplantats aus dem Zwischenwirbelraum, da sie als Anschlag für ein Austreibwerkzeug, beispielsweise einen geschlitzten Hammer, dienen kann.

Die eingangs gestellte Aufgabe wird ferner durch ein Implantatsystem umfassend ein Zwischenwirbelimplantat zum Einsetzen in einen Zwischenwirbelraum zwischen zwei benachbarte Wirbelkörper einer menschlichen oder tierischen Wirbelsäule und ein chirurgisches Instrument zum Entfernen des Zwischenwirbelimplantats aus dem Zwischenwirbelraum, wobei das Zwischenwirbelimplantat zwei Anlegelemente zum Anlegen an die benachbarten Wirbelkörper umfasst, die starr oder gelenkig miteinander verbunden sind, erfindungsgemäß dadurch gelöst, dass mindestens eines der zwei Anlageelemente mindestens ein erstes Kopplungsglied und mindestens ein zweites Kopplungsglied umfasst, dass das chirurgische Instrument eines der oben beschriebenen chirurgischen Instrumente ist und dass das mindestens eine erste Kopplungsglied mit dem mindestens einen ersten Kupplungsglied und dass das mindestens eine zweite Kopplungsglied mit dem mindestens einen zweiten Kupplungsglied in der Kupplungsstellung in Eingriff bringbar sind.

Beispielsweise können die Kopplungsglieder des Zwischenwirbelimplantats durch Ausnehmungen, insbesondere Sacklöcher oder Aussparungen oder Vertiefungen, an einem vorderen oder hinteren Ende des Zwischenwirbelimplantats oder an Teilen desselben ausgebildet sein, beispielsweise an den Anlageelementen. Insbesondere können das Zwischenwirbelimplantat und das Instrument so aufeinander abgestimmt sein, dass das Zwischenwirbelimplantat am Instrument sicher gehalten werden kann. Ferner kann das Instrument auch zum Entfernen unterschiedlicher Zwischenwirbelimplantate verwendet werden, insbesondere auch für Implantate, die nicht speziell auf das Instrument abgestimmt sind.

Die eingangs gestellte Aufgabe wird ferner gelöst durch ein Verfahren zum Entfernen eines Zwischenwirbelimplantats aus einem Zwischenwirbelraum zwischen zwei benachbarten Wirbelkörpern einer menschlichen oder tierischen Wirbelsäule, wobei zunächst mindestens ein erstes Kupplungsglied eines chirurgischen Instruments mit mindestens einem Teil des Zwischenwirbelimplantats in einer Anlegestellung in Eingriff gebracht wird, wobei danach mindestens ein zweites Kupplungsglied in distaler Richtung an dem mindestens einen Teil des Zwischenwirbelimplantats vorbeibewegt wird, wobei der mindestens eine Teil des Zwischenwirbelimplantats zwischen dem mindestens einen ersten Kupplungsglied und dem mindestens einen zweiten Kupplungsglied in der Kupplungsstellung eingeklemmt wird durch eine Bewegung des ersten und des zweiten Kupplungsglied relativ zueinander und wobei der klemmend am Instrument in der Kupplungsstellung gehaltene, mindestens eine Teil des Zwischenwirbelimplantats durch Bewegen des Instruments in proximaler Richtung aus dem Zwischenwirbelraum entfernt wird.

Bei diesem Verfahren wird ganz gezielt das Instrument von der Anlegestellung in die Kupplungsstellung überführt durch eine Bewegung des mindestens einen ersten und des mindestens einen zweiten Kupplungsglieds relativ zueinander.

Vorteilhafterweise wird für das Verfahren eines der oben beschriebenen Instrumente verwendet.

Des weiteren ist es vorteilhaft, wenn für das Verfahren ein Implantatsystem verwendet wird, wie es oben beschrieben wurde.

Die nachfolgende Beschreibung einer bevorzugten Ausführungsform der Erfindung dient im Zusammenhang mit der Zeichnung der näheren Erläuterung. Es zeigen:
- Figur 1:: eine perspektivische Ansicht eines erfindungsgemäßen Implantatsystems vor dem Anlegen des Instruments an ein Zwischenwirbelimplantat;
- Figur 2:: eine perspektivische vergrößerte Ansicht des Implantatsystems aus Figur 1 von schräg oben;
- Figur 3:: eine weitere perspektivische Ansicht des erfindungsgemäßen Implantatsystems von schräg vorne.

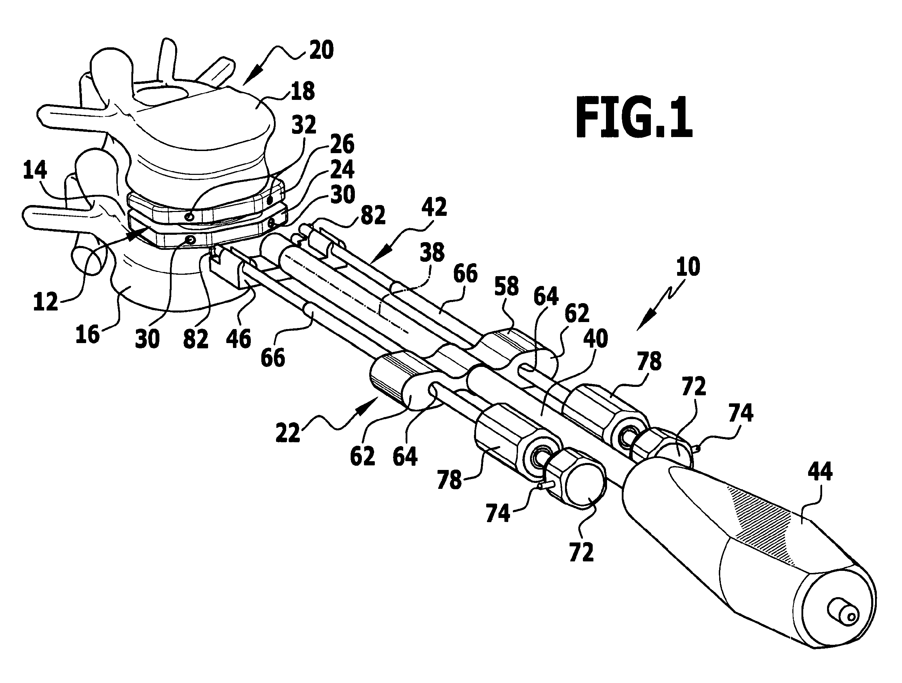

In Figur 1 ist ein insgesamt mit dem Bezugszeichen 10 versehenes, erfindungsgemäßes Implantatsystem dargestellt. Es umfasst ein Zwischenwirbelimplantat 12 und ein chirurgisches Instrument zum Entfernen des Zwischenwirbelimplantats 12 aus einem Zwischenwirbelraum 14 zwischen benachbarten Wirbelkörpern 16 und 18 einer Wirbelsäule 20 in Form eines insgesamt mit dem Bezugszeichen 22 versehenen Revisionsinstruments. Das Zwischenwirbelimplantat 12 ist in Form einer Bandscheibenprothese ausgebildet mit zwei plattenförmigen, jeweils an einem Wirbelkörper 16 beziehungsweise 18 anliegenden Anlageelementen 24 und 26 sowie einem am Anlageelement 24 gelagerten Einsatz 28 mit einer nicht dargestellten sphärischen Gelenkfläche, an der eine korrespondierende Fläche des Anlageelements 26 anliegt, so dass die beiden Anlageelemente 24 und 26 relativ zueinander bewegbar sind und im Wesentlichen ein Kugelgelenk ausbilden.

Das Zwischenwirbelimplantat 12 wird normalerweise so in den Zwischenwirbelraum 14 eingesetzt, dass an den Anlageelementen 24 und 26 jeweils paarweise vorgesehene Kopplungsglieder in Form von Sacklochbohrungen 30 und 32 mit ihren Längsachsen parallel zueinander ausgerichtet sind und in ventraler Richtung weisen. Jedes der Anlageelemente 24 und 26 weist ein weiteres Paar Kopplungsglieder auf, die auf einer dorsalen, also einer Rückseite des jeweiligen Anlageelements 24 beziehungsweise 26 in Form von Vertiefungen 34 beziehungsweise 36 ausgebildet sind. Jede Vertiefung 34 am Anlageelement 24 ist gegenüber einer Vertiefung 36 am Anlageelement 26 angeordnet, wobei die Vertiefungen 34 sowohl in dorsaler Richtung als auch in Richtung auf das jeweilige Anlageelement 26 hin geöffnet sind, die Vertiefungen 36 sowohl in dorsaler Richtung als auch in Richtung auf das jeweilige Anlageelement 24 hin.

Das Revisionsinstrument 22 weist einen eine Längsachse 38 definierenden massiven stabförmigen Schaft 40 auf, welcher an einem distalen Ende einen Kupplungsteil 42 und an einem proximalen Ende einen Handgriff 44 zum Halten des Revisionsinstruments 22 aufweist. Am distalen Ende des Schafts 40 ist eine Endplatte 46 angeordnet, und zwar derart, dass eine Flächennormale der Endplatte 46 senkrecht zur Längsachse 38 verläuft. Die Endplatte 46 ist am Schaft 40 etwas versetzt unterhalb der Längsachse 38 angeordnet und mit einer flachen U-förmigen Ausnehmung 48 versehen, die in distaler Richtung weisend geöffnet ist und eine Implantataufnahme bildet. Benachbart der Ausnehmung 48 weisen zwei Stirnflächen 50 in distaler Richtung, ebenso wie zwei von den Stirnflächen 50 vorstehende, erste Kupplungsglieder bildende zapfenförmige Kupplungsvorsprünge 52, deren Längsachsen parallel zur Längsachse 38 verlaufen. Die Kupplungsvorsprünge 52 sind derart angeordnet, dass sie entweder in die Sacklochbohrungen 30 oder 32 an den Anlageelementen 24 beziehungsweise 26 eintauchen können, und zwar so, bis das jeweilige Anlageelement 24 beziehungsweise 26 an den Stirnflächen 50 anschlägt, die so axiale Anschläge bilden zum Verhindern einer Bewegung des Zwischenwirbelimplantats 12 und des Revisionsinstruments 22 aufeinander zu.

An der Endplatte 46 sind symmetrisch zur Längsachse 38 und bezogen auf den Schaft 40 diametral gegenüberliegend zwei langgestreckte Lagerschalen 54 angeordnet, die jeweils mit einem von der Endplatte 46 weg weisenden, parallel zur Längsachse 38 verlaufenden Längsschlitz 56 versehen sind.

Die Lagerschalen 54 bilden zusammen mit einem Stablager 58 eine Drehlagerung für zwei Schub- und Zugglieder 60. Der Kupplungsteil 42 umfasst die Endplatte 46 mit den daran angeordneten Lagerschalen 54, Stablager 58 sowie die an der so gebildeten Drehlagerung gelagerten Schub- und Zugglieder 60. Das Stablager 58 umfasst zwei massive, seitlich vom Schaft 40 abstehende Lagerböcke 62, die mit parallel zur Längsachse 38 verlaufenden Lagerbohrungen 64 versehen sind. Sie sind koaxial zu den Längsschlitzen 56 ausgerichtet, so dass ein Schaft 66 des Schub- und Zugglieds 60, welcher im Durchmesser deutlich kleiner ist als der Schaft 40, von proximal her kommend durch die Lagerbohrungen 64 hindurchgesteckt und in den jeweiligen Längsschlitz 56 eingeführt werden kann. Alternativ wäre es auch denkbar, den Schaft 66 des Schub- und Zugglieds 60 von distal her kommend durch die Lagerschalen 54 und die Lagerbohrungen 64 einzuführen und anschließend ein proximales Ende des Schafts 66 mit einem Anschlagkörper zu verbinden, beispielsweise durch Aufschrauben oder Verschweißen. Im Bereich zwischen dem Stablager 58 und der Endplatte 46 verjüngt sich ein Querschnitt der Schäfte 66 einstufig in Richtung auf ihr distales Ende hin. An diesem ist jeweils ein quer zu einer Kupplungsgliedlängsachse 68 des Schafts 66 seitlich vom Schaft 66 vorstehender, ein zweites Kupplungsglied bildender Rückhaltevorsprung 70 angeordnet.

An einem proximalen Ende des Schafts 66 ist ein auch als Anschlagkörper dienender Drehknopf 72 angeordnet, von dem in radialer Richtung ein dünner zylindrischer Zapfen 74 absteht, der ein Markierelement bildet, welches in die gleiche Richtung weist, wie der zugehörige Rückhaltevorsprung 70 des Schub- und Zugglieds 60.

In einem Bereich des Schafts 66 zwischen dem Stablager 58 und dem Drehknopf 72 ist ein Gewindeabschnitt 76 vorgesehen, auf den eine in Richtung der Kupplungsgliedlängsachse 68 langgestreckte Kontermutter 78 aufgeschraubt ist, welche einen Anschlag bildet, um eine Relativbewegung des Schub- und Zugglieds 60 und des Stablagers 58 zu begrenzen, und zwar dadurch, dass die Kontermutter mit ihrem distalen Ende 80 an das Stablager 58 anschlägt.

Alternativ zu dem oben beschriebenen Revisionsinstrument 22 sind auch Instrumente denkbar, die prinzipiell nur ein Schub- und Zugglied 60 aufweisen und somit auch nur ein zweites Kupplungsglied. Alternativ wären jedoch auch drei oder mehr entsprechende Schub- und Zugglieder 60 denkbar. Analog kann auch nur ein oder können auch mehr als drei Kupplungsvorsprünge 52, die erste Kupplungsglieder bilden, am Revisionsinstrument vorgesehen sein.

Das Revisionsinstrument 22 ist vorzugsweise bis auf den Handgriff 44 vollständig aus einem Metall hergestellt und sterilisierbar.

Ferner sei angemerkt, dass das Revisionsinstrument 22 insgesamt spiegelsymmetrisch zu einer die Längsachse 38 enthaltenden Symmetrieebene ausgebildet ist.

Die Funktionsweise des Revisionsinstruments 22 und ein Verfahren zum Entfernen des Zwischenwirbelimplantats 12 aus dem Zwischenwirbelraum 14 wird nachfolgend näher beschrieben.

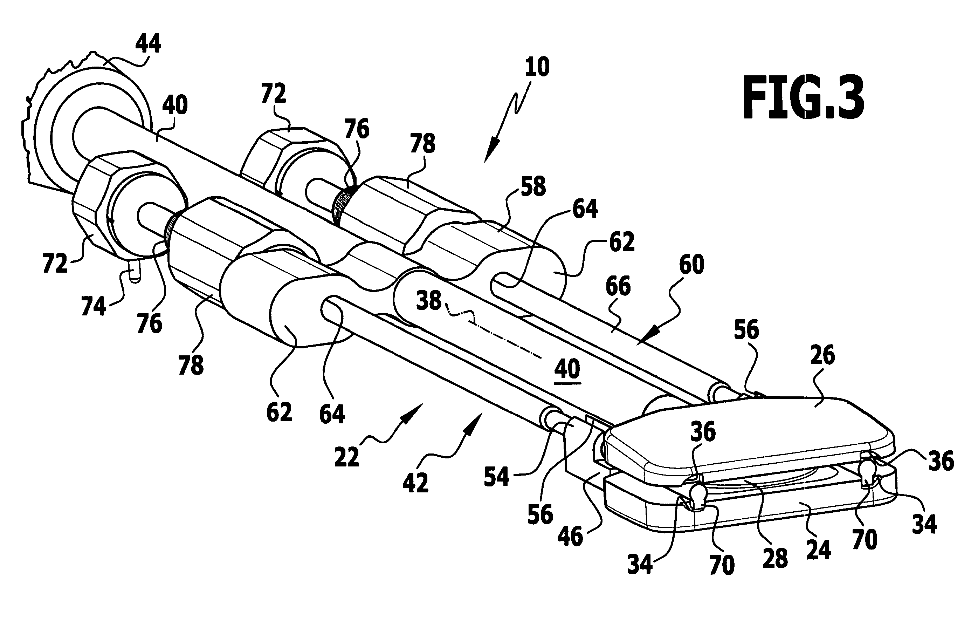

Vorzugsweise werden vor dem Anlegen der Endplatte 46 an eines der Anlageelemente 24 beziehungsweise 26 die beiden Schub- und Zugglieder 60 zurückgezogen, bis die Rückhaltevorsprünge 70 mit einer in proximaler Richtung weisenden Klemmfläche 82 an der Lagerschale 54 anschlagen. Die Rückhaltevorsprünge 70 nehmen somit bezogen auf die Längsachse 38 eine Position proximalseitig der Kupplungsvorsprünge 52 in einer Anlegestellung ein. Die Kupplungsvorsprünge 52 können jetzt auf einfache Weise entweder in die Sacklochbohrungen 30 oder 32 eingeführt werden. In dieser Anlegestellung können nun die Schub- und Zugglieder 60 in distaler Richtung vorgeschoben werden, und zwar derart, dass distale Enden der Schäfte 66 mit den Rückhaltevorsprüngen 70 zwischen den Anlageelementen 24 und 26 hindurch und seitlich am Einsatz 28 vorbei bewegt werden, bis die Rückhaltevorsprünge 70 über eine Rückseite der Anlageelemente 24 und 26 vorstehen. Danach werden die Schub- und Zugglieder 60 um 90° verschwenkt und zurückgezogen, bis die Rückhaltevorsprünge 70 in die Vertiefungen 34 beziehungsweise 36 eintauchen. Alternativ wäre es denkbar, die Schub- und Zugglieder 60 nur so weit vorzuschieben, dass die Rückhaltevorsprünge 70 im Bereich der Vertiefungen 34 und 36 eine Stellung einnehmen, in der durch eine Verdrehung des Schub- und Zugglieds 60 um 90° die Rückhaltevorsprünge 70 in die Vertiefungen 34 beziehungsweise 36 hineinverschwenkt werden können. Die Klemmflächen 82 liegen dann in beiden Fällen an einer in distaler Richtung weisenden Anlagefläche der jeweiligen Vertiefung 34 beziehungsweise 36 an. Das Revisionsinstrument 22 nimmt dann die in Figur 3 dargestellte Kupplungsstellung ein.

Um das Revisionsinstrument 22 an einem der Anlageelemente 24 oder 26 zu sichern, können die Kontermuttern 78 in Richtung auf das Stablager 58 hin verschraubt werden, so dass das betreffende Anlageelement 24 beziehungsweise 26 klemmend zwischen den Stirnflächen 50 und den Klemmflächen 82 gehalten ist.

Durch Zurückziehen des Revisionsinstruments 22 in proximaler Richtung kann eines der Anlageelemente 24 beziehungsweise 26 aus dem Zwischenwirbelraum 14 herausgezogen werden. In analoger Weise kann das im Zwischenwirbelraum 14 verbliebene andere Anlageelement ebenfalls entfernt werden.

Damit auch sehr fest sitzende Zwischenwirbelimplantate 12 aus dem Zwischenwirbelraum 14 entfernt werden können, kann optional beabstandet vom Handgriff 44 und weiter proximalseitig zu diesem an einer Verlängerung des Schafts 40 eine quer zur Längsachse 38 orientierte, nicht dargestellte Endplatte angeordnet sein, so dass mit einem Austreibwerkzeug, beispielsweise einem Hammer oder einem geschlitzten Hammer, gegen diese Endplatte geschlagen und damit das Zwischenwirbelimplantat 12 aus dem Zwischenwirbelraum 14 herausgetrieben werden kann.

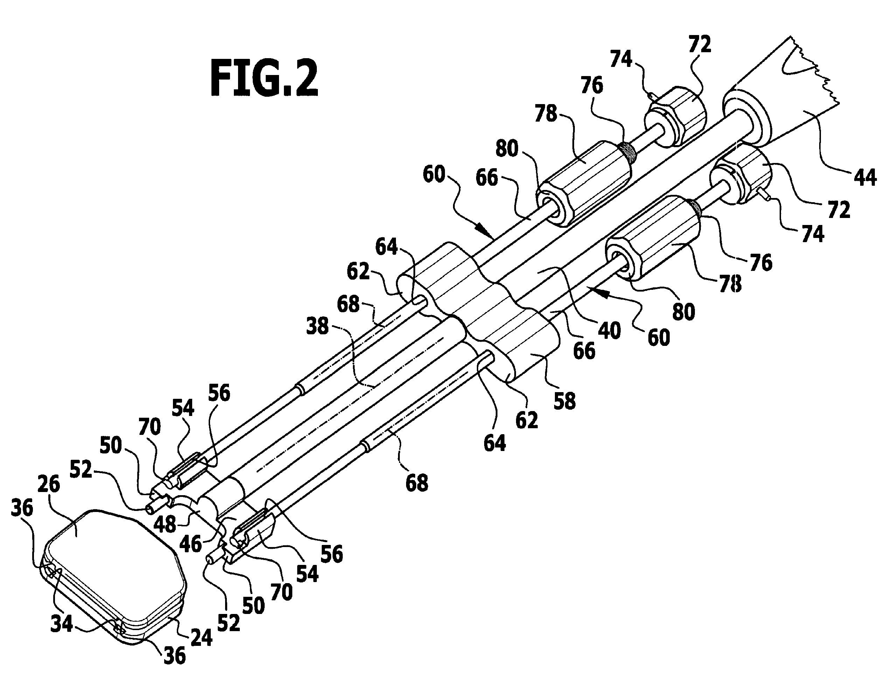

Mit dem erfindungsgemäßen Revisionsinstrument 22 kann nicht nur eine Art von Zwischenwirbelimplantaten 12, wie in den Figuren 1 bis 3 dargestellt, entfernt werden, sondern auch nicht dargestellte Zwischenwirbelimplantate unterschiedlicher Größe. Dies deshalb, weil ein Abstand des Kupplungsvorsprungs 52 und des Rückhaltevorsprungs 70 voneinander aufgrund der relativen Bewegbarkeit des Kupplungsvorsprungs 52 und des Rückhaltevorsprungs 70 veränderbar ist, beispielsweise durch Verschieben des Schub- und Zugglieds in distaler oder proximaler Richtung parallel zur Kupplungsgliedlängsachse 68.

## Patentansprüche

1. Chirurgisches Instrument (22) zum Entfernen eines Zwischenwirbelimplantats (12) aus einem Zwischenwirbelraum (14) einer menschlichen oder tierischen Wirbelsäule (20) umfassend einen am distalen Ende des Instruments (22) angeordneten Kupplungsteil (42) zum Anlegen an das Zwischenwirbelimplantat (12) in einer Anlegestellung, wobei der Kupplungsteil (42) von der Anlegestellung in eine Kupplungsstellung bringbar ist, in welcher das Zwischenwirbelimplantat (12) kraft- und/oder formschlüssig am Kupplungsteil (42) gehalten ist, wobei der Kupplungsteil (42) mindestens ein erstes Kupplungsglied (52) und mindestens ein zweites Kupplungsglied (70) umfasst, welche mit dem Zwischenwirbelimplantat (12) in der Kupplungsstellung in Eingriff bringbar sind, **dadurch gekennzeichnet, dass** das mindestens eine erste Kupplungsglied (52) und das mindestens eine zweite Kupplungsglied (70) relativ zueinander derart bewegbar angeordnet sind, dass ein Abstand des mindestens einen ersten Kupplungsglieds (52) und des mindestens einen zweiten Kupplungsglieds (70) in einer Richtung parallel zu einer vom Instrument (22) definierten Längsachse (38) veränderbar ist zum Überführen des Instruments (22) von der Anlegestellung in die Kupplungsstellung und/oder umgekehrt.

2. Chirurgisches Instrument nach Anspruch 1, **dadurch gekennzeichnet, dass** an einem proximalen Ende des Instruments (22) ein Griffteil (44) vorgesehen ist zum Halten des Instruments (22).

3. Chirurgisches Instrument nach einem der voranstehenden Ansprüche, **dadurch gekennzeichnet, dass** zwei erste Kupplungsglieder (52) vorgesehen sind.

4. Chirurgisches Instrument nach einem der voranstehenden Ansprüche, **dadurch gekennzeichnet, dass** zwei zweite Kupplungsglieder (70) vorgesehen sind.

5. Chirurgisches Instrument nach Anspruch 4, **dadurch gekennzeichnet, dass** die zwei zweiten Kupplungsglieder (70) unabhängig voneinander bewegbar sind.

6. Chirurgisches Instrument nach einem der voranstehenden Ansprüche, **dadurch gekennzeichnet, dass** das mindestens eine erste Kupplungsglied (52) und das mindestens eine zweite Kupplungsglied (70) derart am Instrument (22) angeordnet und gelagert sind, dass sie relativ zueinander parallel oder im Wesentlichen parallel zur Längsachse (38) verschiebbar sind.

7. Chirurgisches Instrument nach einem der voranstehenden Ansprüche, **dadurch gekennzeichnet, dass** das Instrument (22) einen die Längsachse (38) definierenden Schaft (40) umfasst und dass der Kupplungsteil (42) am distalen Ende oder im Bereich des distalen Endes des Schafts angeordnet ist.

8. Chirurgisches Instrument nach Anspruch 7, **dadurch gekennzeichnet, dass** das mindestens eine erste Kupplungsglied (52) am distalen Ende oder im Bereich des distalen Endes des Schafts (40) angeordnet ist.

9. Chirurgisches Instrument nach einem der voranstehenden Ansprüche, **dadurch gekennzeichnet, dass** das mindestens eine erste Kupplungsglied (52) in Form eines in distaler oder im Wesentlichen in distaler Richtung weisenden Kupplungsvorsprungs (52) ausgebildet ist.

10. Chirurgisches Instrument nach einem der voranstehenden Ansprüche, **dadurch gekennzeichnet, dass** am Instrument (22) mindestens ein axialer Anschlag (50) vorgesehen ist zum Begrenzen einer Bewegung des Zwischenwirbelimplantats (12) und des Instruments (22) aufeinander zu.

11. Chirurgisches Instrument nach Anspruch 10, **dadurch gekennzeichnet, dass** der mindestens eine axiale Anschlag (50) eine in distaler oder im Wesentlichen in distaler Richtung weisende Anschlagfläche (50) umfasst, an der das Zwischenwirbelimplantat (12) in der Kupplungsstellung mindestens abschnittsweise flächig anliegen kann.

12. Chirurgisches Instrument nach einem der Ansprüche 10 oder 11, **dadurch gekennzeichnet, dass** der mindestens eine axiale Anschlag (50) benachbart oder im Bereich des mindestens einen ersten Kupplungsglieds (52) angeordnet ist.

13. Chirurgisches Instrument nach einem der Ansprüche 10 bis 12, **dadurch gekennzeichnet, dass** jedem ersten Kupplungsglied (52) ein axialer Anschlag (50) zugeordnet ist.

14. Chirurgisches Instrument nach einem der Ansprüche 10 bis 13, **dadurch gekennzeichnet, dass** das mindestens eine zweite Kupplungsglied (70) eine in Richtung auf den mindestens einen axialen Anschlag (50) hin weisende Klemmfläche (82) umfasst, so dass das Zwischenwirbelimplantat (12) in der Kupplungsstellung zwischen der Klemmfläche (82) und dem mindestens einen axialen Anschlag (50) formschlüssig und/oder klemmend gehalten werden kann.

15. Chirurgisches Instrument nach einem der voranstehenden Ansprüche, **dadurch gekennzeichnet, dass** das Instrument (22) eine die Längsachse (38) enthaltende Symmetrieebene aufweist.

16. Chirurgisches Instrument nach einem der voranstehenden Ansprüche, **dadurch gekennzeichnet, dass** das mindestens eine zweite Kupplungsglied (70) in der Anlegestellung so weit in distaler Richtung bewegbar ist, dass ein Abstand zwischen dem mindestens einen ersten und dem mindestens einen zweiten Kupplungsglied (52, 70) größer ist als in einer Kupplungsstellung, in welcher das Zwischenwirbelimplantat (12) kraft- und/oder formschlüssig zwischen dem mindestens einen ersten und dem mindestens einen zweiten Kupplungsglied (52, 70) gehalten werden kann.

17. Chirurgisches Instrument nach einem der voranstehenden Ansprüche, **dadurch gekennzeichnet, dass** das mindestens eine zweite Kupplungsglied (70) in der Anlegestellung so weit in proximaler Richtung bewegbar ist, dass es bezogen auf die Längsachse (38) eine Position proximalseitig des mindestens einen ersten Kupplungsglieds (52) einnimmt.

18. Chirurgisches Instrument nach einem der voranstehenden Ansprüche, **dadurch gekennzeichnet, dass** das mindestens eine zweite Kupplungsglied (70) in einer Ebene quer zur Längsachse (38) bewegbar ist zum Überführen des Instruments (22) von der Anlegestellung in die Kupplungsstellung.

19. Chirurgisches Instrument nach einem der voranstehenden Ansprüche, **dadurch gekennzeichnet, dass** das mindestens eine zweite Kupplungsglied (70) um eine Kupplungsgliedlängsachse (68), die parallel oder im Wesentlichen parallel zur Längsachse (38) verläuft, verdrehbar gelagert ist.

20. Chirurgisches Instrument nach einem der voranstehenden Ansprüche, **dadurch gekennzeichnet, dass** am Instrument (22) mindestens ein parallel oder im Wesentlichen parallel zur Längsachse (38) verschieblich gelagertes Schub- und Zugglied (60) vorgesehen ist und dass das mindestens eine zweite Kupplungsglied (70) an einem distalen Ende oder im Bereich desselben am Schub- und Zugglied (60) angeordnet ist und quer oder im Wesentlichen quer zur Längsachse (38) von diesem absteht.

21. Chirurgisches Instrument nach Anspruch 20, **dadurch gekennzeichnet, dass** das mindestens eine Schub- und Zugglied (60) eine Kupplungsgiiediängsachse (68) definiert und am instrument (22) um diese verdrehbar gelagert ist.

22. Chirurgisches Instrument nach Anspruch 21, **dadurch gekennzeichnet, dass** am Instrument (22) eine Drehlagerung (54, 58) für das Schub- und Zugglied (60) vorgesehen ist zum Führen einer Verschiebung und/oder einer Verdrehung des Schub- und Zugglieds (60) parallel zur Kupplungsgliedlängsachse (68).

23. Chirurgisches Instrument nach einem der Ansprüche 20 bis 22, **dadurch gekennzeichnet, dass** an einem proximalen Ende des Schub- und Zugglieds (60) ein Griffteil (72) vorgesehen ist.

24. Chirurgisches Instrument nach einem der Ansprüche 20 bis 23, **dadurch gekennzeichnet, dass** das mindestens eine Schub- und Zugglied (60) an seinem proximalen Ende oder im Bereich desselben mindestens ein Markierelement (74) aufweist, welches angibt, in welche Richtung das mindestens eine zweite Kupplungsglied (70) weist.

25. Chirurgisches Instrument nach Anspruch 24, **dadurch gekennzeichnet, dass** das mindestens eine Markierelement (74) ein quer zur Kupplungsgliedlängsachse (68) abstehender Vorsprung (74) ist, der in dieselbe Richtung weisend angeordnet ist wie das mindestens eine zweite Kupplungsglied (70).

26. Chirurgisches Instrument nach einem der Ansprüche 20 bis 25, **dadurch gekennzeichnet, dass** am Instrument ein beweglich gelagerter Anschlag (78) vorgesehen ist zum Begrenzen einer Bewegung des mindestens einen zweiten Kupplungsglieds (70) in distaler Richtung.

27. Chirurgisches Instrument nach Anspruch 26, **dadurch gekennzeichnet, dass** der beweglich gelagerte Anschlag (78) am Schub- und Zugglied (60) angeordnet ist.

28. Chirurgisches Instrument nach Anspruch 27, **dadurch gekennzeichnet, dass** das Schub- und Zugglied (60) einen Gewindeabschnitt (76) umfasst und dass der beweglich gelagerte Anschlag (78) eine zum Gewindeabschnitt (76) korrespondierende und auf diesen aufgeschraubte Mutter (78) umfasst.

29. Chirurgisches Instrument nach Anspruch 26, **dadurch gekennzeichnet, dass** der beweglich gelagerte Anschlag (78) am Schaft (40) angeordnet ist.

30. Chirurgisches Instrument nach einem der voranstehenden Ansprüche, **dadurch gekennzeichnet, dass** an einem proximalen Ende des Instruments (22) eine quer oder im Wesentlichen quer zur Längsachse (38) orientierte Endplatte angeordnet ist.

31. lmplantatsystem (10) umfassend ein Zwischenwirbelimplantat (12) zum Einsetzen in einen Zwischenwirbelraum (14) zwischen zwei benachbarte Wirbelkörper (16, 18) einer menschlichen oder tierischen Wirbelsäule (20) und ein chirurgisches Instrument (22) zum Entfernen des Zwischenwirbelimplantats (12) aus dem Zwischenwirbelraum (14), wobei das Zwischenwirbelimplantat (12) zwei Anlageelemente (24, 26) zum Anlegen an die benachbarten Wirbelkörper (16, 18) umfasst, die starr oder gelenkig miteinander verbunden sind, **dadurch gekennzeichnet, dass** mindestens eines der zwei Anlageelemente (24, 26) mindestens ein erstes Kopplungsglied (30, 32) und mindestens ein zweites Kopplungsglied (34, 36) aufweist, dass das chirurgische Instrument (22) ein Instrument (22) nach einem der voranstehenden Ansprüche ist und dass das mindestens eine erste Kopplungsglied (30, 32) mit dem mindestens einen ersten Kupplungsglied (52) und dass das mindestens eine zweite Kopplungsglied (34, 36) mit dem mindestens einen zweiten Kupplungsglied (70) in der Kupplungsstellung in Eingriff bringbar sind.

## Claims

1. Surgical instrument (22) for removing an intervertebral implant (12) from an intervertebral space (14) of a human or animal spinal column (20), comprising a coupling part (42) arranged at the distal end of the instrument (22) for placement on the intervertebral implant (12) in a placement position, the coupling part (42) being able to be brought from the placement position into a coupling position in which the intervertebral implant (12) is held with force locking and/or positive locking on the coupling part (42), the coupling part (42) comprising at least one first coupling element (52) and at least one second coupling element (70), which are engageable with the intervertebral implant (12) in the coupling position, **characterized in that** the at least one first coupling element (52) and the at least one second coupling element (70) are arranged so as to be movable relative to each other in such a way that a spacing between the at least one first coupling element (52) and the at least one second coupling element (70) in a direction parallel to a longitudinal axis (38) defined by the instrument (22) is alterable for transferring the instrument (22) from the placement position to the coupling position and/or vice versa.

2. Surgical instrument in accordance with claim 1, **characterized in that** a handle (44) is provided at a proximal end of the instrument (22) for holding the instrument (22).

3. Surgical instrument in accordance with any one of the preceding claims, **characterized in that** two first coupling elements (52) are provided.

4. Surgical instrument in accordance with any one of the preceding claims, **characterized in that** two second coupling elements (70) are provided.

5. Surgical instrument in accordance with claim 4, **characterized in that** the two second coupling elements (70) are movable independently of each other.

6. Surgical instrument in accordance with any one of the preceding claims, **characterized in that** the at least one first coupling element (52) and the at least one second coupling element (70) are arranged and mounted on the instrument (22) in such a way that they are displaceable relative to each other parallel or substantially parallel to the longitudinal axis (38).

7. Surgical instrument in accordance with any one of the preceding claims, **characterized in that** the instrument (22) comprises a shaft (40) defining the longitudinal axis (38), and **in that** the coupling part (42) is arranged at the distal end or in the area of the distal end of the shaft.

8. Surgical instrument in accordance with claim 7, **characterized in that** the at least one first coupling element (52) is arranged at the distal end or in the area of the distal end of the shaft (40).

9. Surgical instrument in accordance with any one of the preceding claims, **characterized in that** the at least one first coupling element (52) is in the form of a coupling projection (52) pointing in distal direction or substantially in distal direction.

10. Surgical instrument in accordance with any one of the preceding claims, **characterized in that** at least one axial stop (50) is provided on the instrument (22) for delimiting a movement of the intervertebral implant (12) and the instrument (22) towards each other.

11. Surgical instrument in accordance with claim 10, **characterized in that** the at least one axial stop (50) comprises a stop surface (50) pointing in distal or substantially in distal direction, on which the intervertebral implant (12) is able to bear at least in areas thereof with surface-to-surface contact therewith in the coupling position.

12. Surgical instrument in accordance with claim 10 or 11, **characterized in that** the at least one axial stop (50) is arranged adjacent to or in the area of the at least one first coupling element (52).

13. Surgical instrument in accordance with any one of claims 10 to 12, **characterized in that** an axial stop (50) is associated with each first coupling element (52).

14. Surgical instrument in accordance with any one of claims 10 to 13, **characterized in that** the at least one second coupling element (70) comprises a clamping surface (82) pointing in the direction towards the at least one axial stop (50), so that in the coupling position the intervertebral implant (12) can be held in a positively locked and/or clamped manner between the clamping surface (82) and the at least one axial stop (50).

15. Surgical instrument in accordance with any one of the preceding claims, **characterized in that** the instrument (22) comprises a plane of symmetry containing the longitudinal axis (38).

16. Surgical instrument in accordance with any one of the preceding claims, **characterized in that** in the placement position, the at least one second coupling element (70) is movable so far in distal direction that a spacing between the at least one first coupling element (52) and the at least one second coupling element (70) is greater than in a coupling position in which the intervertebral implant (12) can be held with force locking and/or positive locking between the at least one first coupling element (52) and the at least one second coupling element (70).

17. Surgical instrument in accordance with any one of the preceding claims, **characterized in that** in the placement position, the at least one second coupling element (70) is movable so far in proximal direction that it assumes in relation to the longitudinal axis (38) a position on the proximal side of the at least one first coupling element (52).

18. Surgical instrument in accordance with any one of the preceding claims, **characterized in that** the at least one second coupling element (70) is movable in a plane transversely to the longitudinal axis (38) in order to transfer the instrument (22) from the placement position to the coupling position.

19. Surgical instrument in accordance with any one of the preceding claims, **characterized in that** the at least one second coupling element (70) is mounted for rotation about a coupling element longitudinal axis (68) extending parallel or substantially parallel to the longitudinal axis (38).

20. Surgical instrument in accordance with any one of the preceding claims, **characterized in that** at least one push-and-pull member (60) mounted for displacement parallel or substantially parallel to the longitudinal axis (38) is provided on the instrument (22), and **in that** the at least one second coupling element (70) is arranged at a distal end or in the area thereof on the push-and-pull member (60) and protrudes from the latter transversely or substantially transversely to the longitudinal axis (38).

21. Surgical instrument in accordance with claim 20, **characterized in that** the at least one push-and-pull member (60) defines a coupling element longitudinal axis (68) and is mounted on the instrument (22) for rotation about this coupling element longitudinal axis.

22. Surgical instrument in accordance with claim 21, **characterized in that** a rotary bearing (54, 58) for the push-and-pull member (60) is provided on the instrument (22) for guiding displacement and/or rotation of the push-and-pull member (60) parallel to the coupling element longitudinal axis (68).

23. Surgical instrument in accordance with any one of claims 20 to 22, **characterized in that** a handle portion (72) is provided at a proximal end of the push-and-pull member (60).

24. Surgical instrument in accordance with any one of claims 20 to 23, **characterized in that** the at least one push-and-pull member (60) comprises at its proximal end or in the area thereof at least one marking element (74) which indicates the direction in which the at least one second coupling element (70) points.

25. Surgical instrument in accordance with claim 24, **characterized in that** the at least one marking element (74) is a projection (74) which protrudes transversely to the coupling element longitudinal axis (68) and is arranged so as to point in the same direction as the at least one second coupling element (70).

26. Surgical instrument in accordance with any one of claims 20 to 25, **characterized in that** a movably mounted stop (78) is provided on the instrument for delimiting a movement of the at least one second coupling element (70) in distal direction.

27. Surgical instrument in accordance with claim 26, **characterized in that** the movably mounted stop (78) is arranged on the push-and-pull member (60).

28. Surgical instrument in accordance with claim 27, **characterized in that** the push-and-pull member (60) comprises a threaded section (76), and **in that** the movably mounted stop (78) comprises a nut (78) corresponding to the threaded section (76) and screwed onto the latter.

29. Surgical instrument in accordance with claim 26, **characterized in that** the movably mounted stop (78) is arranged on the shaft (40).

30. Surgical instrument in accordance with any one of the preceding claims, **characterized in that** an end plate oriented transversely or substantially transversely to the longitudinal axis (38) is arranged at a proximal end of the instrument (22).

31. Implant system (10) comprising an intervertebral implant (12) for insertion into an intervertebral space (14) between two adjacent vertebrae (16, 18) of a human or animal spinal column (20), and a surgical instrument (22) for removing the intervertebral implant (12) from the intervertebral space (14), the intervertebral implant (12) comprising two bearing elements (24, 26) for placement on the adjacent vertebrae (16, 18), which are rigidly or articulatedly connected to each other, **characterized in that** at least one of the two bearing elements (24, 26) comprises at least one first coupling member (30, 32) and at least one second coupling member (34, 36), **in that** the surgical instrument (22) is an instrument (22) in accordance with any one of the preceding claims, and **in that** the at least one first coupling member (30, 32) is engageable with the at least one first coupling element (52) and the at least one second coupling member (34, 36) with the at least one second coupling element (70) in the coupling position.

## Revendications

1. Instrument chirurgical (22) pour retirer un implant intervertébral (12) d'une cavité intervertébrale (14) d'une colonne vertébrale humaine ou animale (20) comprenant une partie de couplage (42) disposée à l'extrémité distale de l'instrument (22) pour l'application à l'implant intervertébral (12) dans une position d'application, où la partie de couplage (42) peut être amenée de la position d'application dans une position de couplage dans laquelle l'implant intervertébral (12) est maintenu sur la partie de couplage (42) par assemblage de force et/ou de forme, où la partie de couplage (42) comprend au moins un premier organe de couplage (52) et au moins un deuxième organe de couplage (70), qui peuvent être amenés en prise avec l'implant intervertébral (12) dans la position de couplage, **caractérisé en ce que** le au moins un premier organe de couplage (52) et le au moins un deuxième organe de couplage (70) sont agencés de manière déplaçable l'un par rapport à l'autre de telle manière qu'une distance du au moins un premier organe de couplage (52) et du au moins un deuxième organe de couplage (70) dans une direction parallèle à un axe longitudinal (38) défini par l'instrument (22) est modifiable pour le passage de l'instrument (22) de la position d'application à la position de couplage et/ou inversement.

2. Instrument chirurgical selon la revendication 1, **caractérisé en ce qu'**il est prévu sur une extrémité proximale de l'instrument (22) une partie de préhension (44) pour maintenir l'instrument (22).

3. Instrument chirurgical selon l'une des revendications précédentes, **caractérisé en ce que** deux premiers organes de couplage (52) sont prévus.

4. Instrument chirurgical selon l'une des revendications précédentes, **caractérisé en ce que** deux deuxièmes organes de couplage (70) sont prévus.

5. Instrument chirurgical selon la revendication 4, **caractérisé en ce que** les deux deuxièmes organes de couplage (70) sont déplaçables indépendamment l'un de l'autre.

6. Instrument chirurgical selon l'une des revendications précédentes, **caractérisé en ce que** le au moins un premier organe de couplage (52) et le au moins un deuxième organe de couplage (70) sont disposés et montés sur l'instrument (22) de telle manière qu'ils sont déplaçables l'un par rapport à l'autre parallèlement ou sensiblement parallèlement à l'axe longitudinal (38).

7. Instrument chirurgical selon l'une des revendications précédentes, **caractérisé en ce que** l'instrument (22) comprend une tige (40) définissant l'axe longitudinal (38) et **en ce que** l'organe de couplage (42) est disposé à l'extrémité distale ou dans le domaine de l'extrémité distale de la tige.

8. Instrument chirurgical selon la revendication 7, **caractérisé en ce que** le au moins un premier organe de couplage (52) est disposé à l'extrémité distale ou dans le domaine de l'extrémité distale de la tige (40).

9. Instrument chirurgical selon l'une des revendications précédentes, **caractérisé en ce que** le au moins un premier organe de couplage (52) est agencé sous forme d'une protubérance de couplage (52) tournée dans la direction distale ou sensiblement dans la direction distale.

10. Instrument chirurgical selon l'une des revendications précédentes, **caractérisé en ce qu'**il est prévu sur l'instrument (22) au moins une butée axiale (50) pour limiter un mouvement de l'implant intervertébral (12) et de l'instrument (22) l'un vers l'autre.

11. Instrument chirurgical selon la revendication 10, **caractérisé en ce que** la au moins une butée axiale (50) comprend une surface de butée (50) tournée dans la direction distale ou sensiblement dans la direction distale, sur laquelle peut s'appuyer au moins par segments suivant une surface l'implant intervertébral (12) dans la position de couplage.

12. Instrument chirurgical selon l'une des revendications 10 ou 11, **caractérisé en ce que** la au moins une butée axiale (50) est disposée au voisinage ou dans le domaine du au moins un premier organe de couplage (52).

13. Instrument chirurgical selon l'une des revendications 10 à 12, **caractérisé en ce qu'**une butée axiale (50) est associée à chaque premier organe de couplage (52).

14. Instrument chirurgical selon l'une des revendications 10 à 13, **caractérisé en ce que** le au moins un deuxième organe de couplage (70) comprend une surface de serrage (82) tournée dans la direction de la au moins une butée axiale (50), de sorte que l'implant intervertébral (12) peut être maintenu dans la position de couplage entre la surface de serrage (82) et la au moins une butée axiale (50) par assemblage de forme et/ou par serrage.

15. Instrument chirurgical selon l'une des revendications précédentes, **caractérisé en ce que** l'instrument (22) comprend un plan de symétrie contenant l'axe longitudinal (38).

16. Instrument chirurgical selon l'une des revendications précédentes, **caractérisé en ce que** le au moins un deuxième organe de couplage (70) est déplaçable dans la position d'application en direction distale dans une mesure telle qu'une distance entre le au moins un premier et le au moins un deuxième organe de couplage (52, 70) est plus grande que dans une position de couplage dans laquelle l'implant intervertébral (12) peut être maintenu par assemblage de force et/ou par assemblage de forme entre le au moins un premier et le au moins un deuxième organe de couplage (52, 70).

17. Instrument chirurgical selon l'une des revendications précédentes, **caractérisé en ce que** le au moins un deuxième organe de couplage (70) est déplaçable dans la position d'application en direction proximale dans une mesure telle qu'il occupe par rapport à l'axe longitudinal (38) une position du côté proximal du au moins un premier organe de couplage (52).

18. Instrument chirurgical selon l'une des revendications précédentes, **caractérisé en ce que** le au moins un deuxième organe de couplage (70) est déplaçable dans un plan transversal à l'axe longitudinal (38) pour le passage de l'instrument (22) de la position d'application dans la position de couplage.

19. Instrument chirurgical selon l'une des revendications précédentes, **caractérisé en ce que** le au moins un deuxième organe de couplage (70) est monté de manière à pouvoir tourner autour d'un axe longitudinal d'organe de couplage (68) qui s'étend parallèlement ou sensiblement parallèlement à l'axe longitudinal (38).

20. Instrument chirurgical selon l'une des revendications précédentes, **caractérisé en ce qu'**il est prévu sur l'instrument (22) au moins un organe de poussée et de traction (60) monté de manière déplaçable parallèlement ou sensiblement parallèlement à l'axe longitudinal (38) et **en ce que** le au moins un deuxième organe de couplage (70) est disposé à une extrémité distale ou dans le domaine de celle-ci sur l'organe de poussée et de traction (60) et s'écarte de celui-ci transversalement ou sensiblement transversalement par rapport à l'axe longitudinal (38).

21. Instrument chirurgical selon la revendication 20, **caractérisé en ce que** le au moins un organe de poussée et de traction (60) définit un axe longitudinal d'organe de couplage (68) et est monté sur l'instrument (22) de manière à pouvoir tourner autour de celui-ci.

22. Instrument chirurgical selon la revendication 21, **caractérisé en ce qu'**il est prévu sur l'instrument (22) un logement de rotation (54, 58) pour l'organe de poussée et de traction (60) et pour le guidage d'un déplacement et/ou d'une rotation de l'organe de poussée et de traction (60) parallèlement à l'axe longitudinal d'organe de couplage (68).

23. Instrument chirurgical selon l'une des revendications 20 à 22, **caractérisé en ce qu'**il est prévu sur une extrémité proximale de l'organe de poussée et de traction (60) une partie de préhension (72).

24. Instrument chirurgical selon l'une des revendications 20 à 23, **caractérisé en ce que** le au moins un organe de poussée et de traction (60) comprend à son extrémité proximale ou dans le domaine de celle-ci au moins un élément marqueur (74) qui indique dans quelle direction est tourné le au moins un deuxième organe de couplage (70).

25. Instrument chirurgical selon la revendication 24, **caractérisé en ce que** le au moins un élément marqueur (74) est une protubérance (74) qui s'écarte transversalement de l'axe longitudinal d'organe de couplage (68) qui est disposée en étant tournée dans la même direction que le au moins un deuxième organe de couplage (70).

26. Instrument chirurgical selon l'une des revendications 20 à 25, **caractérisé en ce qu'**il est prévu sur l'instrument une butée (78) montée de manière mobile pour limiter un mouvement du au moins un deuxième organe de couplage (70) en direction distale.

27. Instrument chirurgical selon la revendication 26, **caractérisé en ce que** la butée (78) montée de manière mobile est disposée sur l'organe de poussée et de traction (60).

28. Instrument chirurgical selon la revendication 27, **caractérisé en ce que** l'organe de poussée et de traction (60) comprend un segment fileté (76) et **en ce que** la butée (78) montée de manière mobile comprend un écrou correspondant au segment fileté (76) et vissée sur celui-ci.

29. Instrument chirurgical selon la revendication 26, **caractérisé en ce que** la butée (78) montée de manière mobile est disposée sur la tige (40).

30. Instrument chirurgical selon l'une des revendications précédentes, **caractérisé en ce qu'**une plaque d'extrémité orientée transversalement ou sensiblement transversalement à l'axe longitudinal (38) est disposée à une extrémité proximale de l'instrument (22).

31. Système d'implant (10) comprenant un implant intervertébral (12) destiné à être inséré dans une cavité intervertébrale (14) entre deux vertèbres voisines (16, 18) d'une colonne vertébrale humaine ou animale (20) et un instrument chirurgical (22) pour retirer l'implant intervertébral (12) de la cavité intervertébrale (14), où l'implant intervertébral (12) comprend deux éléments d'application (24, 26) pour l'application sur les vertèbres voisines (16, 18), qui sont reliés entre eux de manière rigide ou articulée, **caractérisé en ce qu'**au moins l'un des deux éléments d'application (24, 26) comprend au moins un premier organe d'accouplement (30, 32) et au moins un deuxième organe d'accouplement (34, 36), **en ce que** l'instrument chirurgical (22) est un instrument (22) selon l'une des revendications précédentes et **en ce que** le au moins un premier organe d'accouplement (30, 32) peut être amené en prise avec le au moins un premier organe de couplage (52) et **en ce que** le au moins un deuxième organe d'accouplement (34, 36) peut être amené en prise avec le au moins un deuxième organe de couplage (70) dans la position de couplage.
